# EUROPÄISCHE PATENTSCHRIFT

(11) **EP 1 324 738 B1**
(45) Veröffentlichungstag und Bekanntmachung des Hinweises auf die Patenterteilung: **20.08.2008**
(21) Anmeldenummer: 01986593.0
(22) Anmeldetag: 05.10.2001
(51) Int. Cl.: A61Q 1/10, A61K 8/81, A61K 8/86

(54) **STRETCH-MASCARA**
STRETCH-MASCARA
MASCARA EXTENSIBLE

(30) Priorität: 13.10.2000 DE 10053052
(43) Veröffentlichungstag der Anmeldung: 09.07.2003
(73) Patentinhaber: Coty B.V., 2031 CC Haarlem (NL)
(72) Erfinder: MATEU, Juan, R., Milton, NJ 07438 (US); MACCHIO, Ralph, Sparta, NJ 07971 (US); KULKARNI, Rupali, A., Bridgewater, NJ 08807 (US); CERNASOV, Dominica, Ringwood, NJ 07456 (US)
(74) Vertreter: Walter, Wolf-Jürgen
(86) Internationale Anmeldenummer: PCT/EP2001/011504
(87) Internationale Veröffentlichungsnummer: WO 2002/030368

(56) Entgegenhaltungen:
- FR-A- 2 659 011
- US-A- 5 620 693

## Beschreibung

Die Erfindung betrifft ein neues als "Stretch-Mascara" bezeichnetes kosmetisches Produkt mit besonderen Dehneigenschaften.

Aus der WO99/22711 ist eine wasserfeste Mascarazusammensetzung bekannt auf Basis eines Stryol-Ethylen-Propylen-Copolymeren als Gelierungsmittel und bei dem u.a. auch Polyethylen-Wachs als filmbildendes Mittel eingesetzt werden kann. Man erhält damit Mascara-Zusammensetzungen mit den üblichen Viskositätswerten von etwa 1,500,000 Pa·s (cP).

Der Erfindung liegt die Aufgabe zugrunde, Mascara-Zusammensetzungen mit niedrigeren Viskositäten und sehr guter Flexibilität und Dehnbarkeit als Wimperntusche auf den Augenlidern zu entwickeln.

Erfindungsgemäß ist das neue Stretch-Mascara auf Basis einer ölphase und einer wäßrige Phase dadurch gekennzeichnet, daß
a) die ölphase 2 bis 10 Gew-%, bezogen auf das Gewicht der ölphase, eines unverzweigten Polyethylenwachses mit einem Molekulargewicht im Bereich von 400 bis 1500 Dalton enthält, und wenigstens ein weiteres Wachs oder öl oder ein Gemisch davon;
b) die wäßrige Phase 0,5 bis 5 Gew-% eines Filmbildners für die wäßrige Phase enthält, ausgewählt aus der Gruppe, die aus PVP/PVP-VA, Vinylcaprolactam/Vinylpyrrolidon/qaternisiertes Vinylimidazol (Polyquaternium-46) und Gemischen davon besteht, wobei die Prozente auf das Gesamtgewicht des Mascara bezogen sind;
c) zusätzlich 0,5 bis 20 Gew-% einer äußeren Filmbildnerphase mit einem wasserlöslichen Filmbildner enthalten sind, ausgewählt aus der Gruppe, die aus PEG/PPG-25/25 Dimethicone/Acrylates/t-Butyl Acrylates Copolymer, Polyurethane-1 und Gemischen davon besteht, wobei die Prozente auf das Gesamtgewicht des Mascara bezogen sind; und
d) weitere kosmetische Pigmente, Träger- und Hilfsstoffe bis 100 Gew-%, enthalten sind.

Die ölphase kann weiterhin natürliche Wachse, andere synthetische Wachse, Weichmacher, höhere C₁₂-C₂₀-Fettsäureester, Emulgatoren, Schutzmittel und Gemische davon enthalten. Der Anteil der ölphase liegt im Bereich von 10 bis 40 Gew-%, bezogen auf das Gewicht des Mascara.

In der ölphase ist das unverzweigte Polyethylen mit einer Molekulargewichtsverteilung von 400 bis 1500 Dalton insbesondere ein solches mit einem Molekulargewicht von 400 bis 600 Dalton. Damit erhält man ein besonders flexibles Produkt. Das Polyethylen hat einen Schmelzpunkt von 75-99 °C und einem Penetrationswert von 5-15 dyn/mm bei 25 °C.

Die Messung des Penetrationswertes erfolgt mit einem Penetrometer , z.B. Lab-Line #4101, Nadel D1321 (hergestellt von Lab-Line Instruments Inc., USA). Eine Probe wird auf ihre Gießtemperatur erhitzt, in ein 250 ml Becherglas bis zu dessen Rand gegossen, abgedeckt und 12-24 Stunden in kontrollierter Umgebung bei 20 °C stehen gelassen. Nach Abziehen der Abdekkung von dem Becherglas erfolgen die Messungen. Die Meßnadel wird am Penetrometerkopf befestigt, erforderlichenfalls wird ein Gewicht am Belastungsstab angebracht, und der Meßkopf wird abgesenkt, so daß die Spitze der Nadel die Probenoberfläche berührt. Danach wird der Kolben vorsichtig freigegeben, und man läßt den Nadelkonus für 5 Sekunden in die Probe eindringen. Der sich ergebende Meßwert wird aufgezeichnet. Vier Werte werden an unterschiedlichen Stellen der Probe gemessen und der Durchschnittswert ermittelt.

Der Penetrationswert von 5 bis 15 dyn/mm bei 25 °C widerspiegelt die Flexibilität und die Weichheit dieses Wachses.

Die weiteren Wachse sind vorzugsweise ausgewählt unter Candelilla-Wachs, Bienenwachs, Stearinsäure, Glycerylmonostearat und Gemischen davon. Besonders bevorzugte Bereiche sind 1 bis 6 Gew-% Candelilla-Wachs, 1-17 Gew-% Bienenwachs, 3-8 Gew-% Stearinsäure, 1-5 Gew-% Glycerylmonostearat. Es können jedoch auch andere Wachse verwendet werden, wie Carnaubawachs, Wollwachs, Hartparaffin, Ceresin, Ozokerit, Silicone und Gemische davon.

Der Anteil des Polyethylens beträgt 2 bis 10 Gewichts-%, bezogen auf das Gewicht der ölphase.

Ein besonders vorteilhaftes Polyethylen ist Performalene 400^{®} (New Phase Technology, Piscataway, NJ, USA) das keine Verzweigungen enthält und damit ein sehr flexibles Polyethylen ist.

Ein weiteres bevorzugtes Merkmal der Erfindung besteht darin, daß die ölphase insgesamt einen Penetrationswert von 5-20 dyn/mm bei 25 °C hat. Das bedeutet, daß das in der ölphase in den meisten Fällen vorliegende Gemisch von Polyethylen, weiteren Wachsen, Estern, Emulgatoren usw. einen Penetrationswert in diesem Bereich aufweist. '

Als Emulgatoren können beispielsweise Sorbitanfettsäureester, Ester von C₁₂-C₂₂-Fettsäuren und Glycerin, Polyglycerin, Pentaerythrit, Zuckeralkohole (z.B. Sorbit), Polyglucoside (z.B. Cellulose); Polyalkylenglycole; Wollwachsalkohole verwendet werden. Vorzugsweise werden 0,2-2 Gew-% Sorbitanoleat eingesetzt.

Die äußeren Filmbildner Polyurethane-1 oder PEG/PPG-25/25 Dimethicone/Acrylates Copolymer (Luviflex Silk^{®}) oder ein Gemisch von beiden werden sonst nahezu ausschließlich nur in Haarsprays verwendet. Im vorliegenden Fall ist der Terpolymer-Filmbildner aus t-Butylacrylat, Methacrylsäure und Dimethicone Copolyol vorteilhaft in einem Anteil von 0,5 bis 10 Gew-%, bezogen auf die Gesamtmasse der Mascara-Zusammensetzung, vorhanden.

Die wäßrige Phase des erfindungsgemäßen Mascara enthält neben dem Filmbildner PVP/PVP-VA, Vinylcaprolactam/Vinylpyrrolidon/quaternisiertes Vinylimidazol (Polyquaternium-46) und Gemischen davon weiterhin Verdickungsmittel, Hydroxyethylcellulose, Neutralisationsmittel, Triethanolamin.

Weiterhin enthält das Stretch-Mascara organische und anorganische Pigmente, Pigmentgemische oder Pulver mit pigmentartiger Wirkung, worunter auch solche mit Perlglanz-Effekt zu verstehen sind. Diese können zum Beispiel umfassen Eisenoxide, natürliche Aluminiumsilicate wie Ocker, Titan(di)oxid, Glimmer, Kaolin, manganhaltige Tone wie Umbra und roter Bolus, Calciumcarbonat, Talkum, Glimmer-Titanoxid, Glimmer-Titanoxid-Eisenoxid und Gemische davon. Der prozentuale Anteil der färbenden Pigmente oder Gemische davon kann im Bereich von 7 bis 15 Gew-% liegen.

Weitere vorteilhafte Bestandteile des erfindungsgemäßen Stretch-Mascara können Haar-Konditionierungsmittel sein, wie Vitamin E oder Vitamin E-acetat im Bereich von 0,1 bis 2 Gew-% und/oder Panthenol im Bereich von 0,1 bis 1,2 Gew-%. Als besonders vorteilhaften Zusatzstoff kann das Mascara 0,1 bis 4 Gew-%, vorzugsweise 0,01 bis 2 Gew-% eines wäßrigen Extraktes der Alge Rhodophycea als Bioextender enthalten, wodurch eine verbesserte Wirksamkeit im Hinblick auf Festigkeit von Haaren und Verbesserung des natürlichen Aussehens erreicht wird.

Es können auch Antioxidationsmittel, wie Vitamin C und Derivate davon, beispielsweise Ascorbylacetate, -phosphate und - palmitate; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Carotinoide und Carotine enthalten sein.

Auch der Zusatz von UV-Filtern, sowohl wasserlöslichen als auch öllöslichen UVA- oder UVB-Filter oder beiden ist vorteilhaft. Zu bevorzugten öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher. Bevorzugte öllösliche UV-Filter sind Benzophenone-3, Butyl-Methoxybenzoylmethane, Octyl Methoxycinnamate, Octyl Salicylate, 4-Methylbenzylidene Camphor, Homosalate und Octyl Dimethyl PABA.

Ein Zusatz von Feuchthaltemitteln, wie Propylenglycol, Butylenglycol, Glycerin oder Gemischen davon kann vorteilhaft sein.

Als Erweichungsmittel (Emollients) kann beispielsweise Panthenol eingesetzt werden, wobei die Konzentration im Bereich von 0,1 bis 1 Gew-% liegen sollte. Auch andere Erweichungsmittel wie Stearate oder Palmitate können verwendet werden.

Insgesamt wird im Gegensatz zu üblichen marktgängigen Mascara-Zusammensetzungen durch das spezielle Polyethylen und die Kombination von inneren und äußeren Filmbildnern eine bedeutender "Dehnungs"-effekt nach dem Auftragen der Zusammensetzung bewirkt. Dieser Streckungs- oder Dehnungseffekt auf den Wimpern führt dazu, daß die Haltbarkeit deutlich verlängert wird und ein sehr guter Kontakt mit dem darunter liegenden Gewebe bzw. Haar erreicht wird. Darüber hinaus wird das Umbiegen der Wimpern erleichtert, und die Wimpern erscheinen länger und voluminöser. Die Zusammensetzung läßt sich verschmierungsfrei und flockenfrei auftragen und verbleibt in diesem Zustand. Es tritt kein Verkleben der Augenlider auf, und Pigmente lassen sich sehr gut in der Emulsion verteilen und damit auch auf den Augenlidern.

Das Mascara kann mit Wasser und Seife leicht entfernt werden.

Ein weitere vorteilhaftes Merkmal des Stretch-Mascara nach der Erfindung besteht darin, daß es eine Viskosität im Bereich von 50.000 bis 500.000 Pa·s (cP) hat, gemessen nach der Brookfield-Methode mit den Spindeln TC/TD/TE bei 25 °C und im Bereich von 50-75 % der Spindelgeschwindigkeit.

Infolge dieser niedrigen Viskosität im Vergleich zu Produkten des Standes der Technik mit ca. 1,500,000 Pa·s (cP) hat das Mascara eine cremige Konsistenz, ist in dieser Konsistenz über einen wesentlich längeren Zeitraum lagerfähiger als übliche Produkte und zeigt darüber hinaus eine deutlich erhöhte Verbrauchszeit nach öffnung und Benutzung durch den Verbraucher.

Die Erfindung betrifft auch ein Verfahren zur Herstellung eines Stretch-Mascara, das dadurch gekennzeichnet ist, daß nach Vermischen der öl- und der Wasserphase die äußere Filmbildnerphase zugesetzt wird, wobei die Zugabe von PEG/PPG-25/25 Dimethicone/Acrylates/t-Butyl Acrylates Copolymer bei 40-76 °C, insbesondere 70-76 °C und portionsweise erfolgt. Nur durch diese Verfahrensweise ist es möglich, eine starke Viskositätserhöhung und einen gummiartigen Charakter des Gemisches zu vermeiden.

Die Erfindung soll nachstehend durch Beispiele näher erläutert werden. Alle Angaben erfolgen in Gewichtsprozent, sofern nichts anderes angegeben ist.

| Beispiele 1-4 **Mascara I-IV** | 1 | 2 | 3 | 4 |
|---|---|---|---|---|
| Polyethylene | 1,2 | 0,5 | 2 | 3 |
| Candelilla wax | | | | |
| (Euphorbia cerifera) | 2,4 | 3 | 1 | 4 |
| Stearic acid | 5,5 | 4,6 | 4 | 5 |
| Glyceryl monostearate | 3,5 | 3 | 3 | 3,3 |
| Beeswax | 2,7 | 3 | 2 | 4 |
| Sorbitan Sesquioleate | 0,6 | 0,6 | 0,4 | 0,4 |
| Polybutene | 0,3 | 0,1 | 0,5 | - |
| Propylparaben | 0,2 | 0,2 | 0,2 | 0,3 |
| Hydroxyethyl cellulose | 0,3 | 0,5 | 0,2 | 0,5 |
| PVP (Polyquaternium-46) | 2,5 | 2,7 | 3 | 3,1 |
| Triethanolamine | 1,1 | 1,5 | 0,8 | 1 |
| Black Iron Oxide | 12 | 13 | 11 | 5 |
| Wasser | ad 100 | ad 100 | ad 100 | ad100 |
| Cellulose | 1 | 0 | 2 | - |
| Polyurethane-1 | 8 | 9 | 13 | 15 |
| PEG/PPG-25/25dimeticone/ | | | | |
| acrylates/t-butyl acrylates | | | | |
| (Luviflex" Silk) | 1,2 | 2 | 1 | 1,8 |
| Methylparaben | 0,2 | 0,2 | 0,2 | 0,4 |
| Tocopheryl Acetate | 0,2 | 0,5 | 1 | 0,5 |
| Panthenol 50% AQ. | 0,2 | 1 | 0,7 | 0,6 |
| Bioextender/ Water hydrolyzed | | | | |
| Rhodyphycea extract | 0,4 | 0,8 | 1,7 | - |
| Phenonip (Parabengemisch) | 0,3 | 0,2 | 0,3 | 0,3 |

Es wurde Polyethylen mit folgenden Parametern in den Beispielen 1, 2 und 3 eingesetzt:
Molekulargewicht 500, Schmelzpunkt 88 °C, Penetrationswert 7. Im Beispiel 4: MG 400, Smp. 79,5, Penetrationswert 15.

Zuerst wurden alle Wachse, Stearinsäure, Polyethylen, Sorbitansesquioleat und Propylparaben auf etwa 95 °C erhitzt und bis zum Erhalt einer klaren Flüssigkeit gerührt. Separat wurde Wasser erwärmt, die Cellulose eingerührt, die Temperatur auf etwa 65 °C erhöht und PVP eingerührt sowie Eisenoxid und Methylparaben. Bei etwa 85 °C wurde die ölphase bis zum Erreichen einer Emulsion der Wasserphase zugesetzt. Danach wurde Luviflex Silk bei etwa 74-76 °C in kleinen Portionen und unter Rühren hinzugegeben, um ein gummiartiges Gemisch zu vermeiden. Anschließend wurden nach der Zugabe von Polyurethane-1 bei etwa 65 °C und weiterer Temperaturabsenkung auf etwa 45 °C die restlichen Bestandteile hinzugegeben und das Gemisch bis zum Erhalt einer cremigen Konsistenz homogenisiert.

## Patentansprüche

1. Stretch-Mascara, enthaltend eine ölphase und eine wäßrige Phase, **dadurch gekennzeichnet, daß**
a) die ölphase 2 bis 10 Gew-%, bezogen auf das Gewicht der ölphase, eines unverzweigten Polyethylenwachses mit einem Molekulargewicht im Bereich von 400 bis 1500 Dalton enthält, und wenigstens ein weiteres Wachs oder öl oder ein Gemisch davon;
b) die wäßrige Phase 0,5 bis 5 Gew-% eines Filmbildners für die wäßrige Phase enthält, ausgewählt aus der Gruppe, die aus PVP/PVP-VA, Vinylcaprolactam/Vinylpyrrolidon/qaternisiertes Vinylimidazol (Polyquaternium-46) und Gemischen davon besteht, wobei die Prozente auf das Gesamtgewicht des Mascara bezogen sind;
c) zusätzlich 0,5 bis 20 Gew-% einer äußeren Filmbildnerphase mit einem wasserlöslichen Filmbildner enthalten sind, ausgewählt aus der Gruppe, die aus PEG/PPG-25/25 Dimethicone/Acrylates/t-Butyl Acrylates Copolymer, Polyurethane-1 und Gemischen davon besteht, wobei die Prozente auf das Gesamtgewicht des Mascara bezogen sind; und
d) weitere kosmetische Pigmente, Träger- und Hilfsstoffe bis 100 Gew-%, enthalten sind.

2. Stretch-Mascara nach Anspruch 1, **dadurch gekennzeichnet, daß** die ölphase einen Nadel-Penetrationswert von 5-20 dyn/mm bei 25 °C hat.

3. Mascara-Zusammensetzung nach Anspruch 1, **dadurch gekennzeichnet, daß** das unverzweigte Polyethylenwachs ein Molekulargewicht im Bereich von 400 bis 600 Dalton hat.

4. Stretch-Mascara nach Anspruch 1, **dadurch gekennzeichnet, daß** die ölphase weiterhin natürliche Wachse, synthetische Wachse, Weichmacher, höhere C₁₂-C₂₀-Fettsäureester, Emulgatoren, Schutzmittel und Gemische davon enthält.

5. Stretch-Mascara nach Anspruch 4, **dadurch gekennzeichnet, daß** die Wachse ausgewählt sind unter Candelilla-Wachs, Bienenwachs, Stearinsäure, Glycerylmonostearat und Gemischen davon.

6. Stretch-Mascara nach Anspruch 1, **dadurch gekennzeichnet, daß** der Anteil der ölphase 10 bis 40 Gew-% beträgt, bezogen auf das Gesamtgewicht des Mascara.

7. Stretch-Mascara nach Anspruch 1, **dadurch gekennzeichnet, daß** der Anteil von PEG/PPG-25/25 Dimethicone/Acrylacrylates/t-Butyl Acrylates Copolymer 0,5 bis 10 Gew-% beträgt, bezogen auf das Gesamtgewicht des Mascara.

8. Stretch-Mascara nach Anspruch 1, **dadurch gekennzeichnet, daß** die wäßrige Phase 0,01 bis 2 Gew-% eines wäßrigen Extraktes der Alge Rhodophycea enthält.

9. Stretch-Mascara nach Anspruch 1, **dadurch gekennzeichnet, daß** es eine Viskosität im Bereich von 50.000 bis 500.000 Pa·s hat, gemessen nach der Brookfield-Methode mit den Spindeln TC/TD/TE bei 25 °C.

10. Verfahren zur Herstellung eines Stretch-Mascara, **dadurch gekennzeichnet, daß** nach Vermischen der öl- und der Wasserphase die äußere Filmbildnerphase zugesetzt wird, wobei die Zugabe von PEG/PPG-25/25 Dimethicone/Acrylates/t-Butyl Acrylates Copolymer bei 40-76 °C und portionsweise erfolgt.

## Claims

1. Stretch mascara containing an oil phase and an aqueous phase, **characterized in that**
a) the oil phase comprises 2 to 10 % by weight, related to the weight of the oil phase, of an unbranched polyethylene wax having a molecular weight ranging between 400 and 1,500 Dalton, and at least one further oil or wax or mixture thereof;
b) the water phase comprises 0.5 to 5 % by weight of a film-forming agent for the water phase selected from the group consisting of PVP/PVP-VA, Vinylcaprolactam/Vinylpyrrolidone/quaternized Vinylimidazol (Polyquaternium-46) and mixtures thereof, the % by weight being related to the total weight of the mascara;
c) additionally 0.5 to 20 % by weight of an external film-forming phase is comprised with a water soluble film-forming agent selected from the group consisting of PEG/PPG-25/25 Dimethicone/Acrylates/t-Butyl Acrylates Copolymer, Polyurethane-1 and mixtures thereof, the % by weight being related to the total weight of the mascara; and
d) further cosmetic pigments, carriers and auxiliary substances up to 100 % by weight are comprised.

2. Stretch mascara according to claim 1, **characterized in that** the oil phase has a needle penetration value of 5-20 dyn/mm at 25 °C.

3. Stretch mascara according to claim 1, **characterized in that** the unbranched polyethylene wax has a molecular weight ranging between 400 and 600 Dalton.

4. Stretch mascara according to claim 1, **characterized in that** the oil phase further comprises natural waxes, synthetic waxes, emollients, higher C₁₂-C₂₀ fatty acid esters, emulsifying agents, protective agents and mixtures thereof.

5. Stretch mascara according to claim 4, **characterized in that** the waxes are selected among candelilla wax, beeswax, stearic acid, glyceryl monostearate and mixtures thereof.

6. Stretch mascara according to Claim 1, **characterized in that** the share of the oil phase is 10 to 40 weight percent relative to the total weight of the mascara.

7. Stretch mascara according to claim 1, **characterized in that** the share of PEG/PPG-25/25 Dimethicone/Acrylates/t-Butyl Acrylates Copolymer is 0.5 to 10 weight percent relative to the total weight of the mascara.

8. Stretch mascara according to claim 1, **characterized in that** the aqueous phase comprises 0,01 to 2 weight percent of an aqueous extract of the rhodophycea alga.

9. Stretch mascara according to claim 1, **characterized in that** it has a viscosity ranging from 50,000 to 500.000 Pa·s, measured according to the Brookfield method using spindles TC/TD/TE at 25 °C.

10. Method for manufacturing a stretch mascara, **characterized in that** after mixing the oil and water phases the external film-formig phase is added, where PEG/PPG-25/25 Dimethicone/Acrylates/t-Butyl Acrylates Copolymer is added in portions at 74-76 °C.

## Revendications

1. Mascara extensible, contenant une phase huileuse et une phase aqueuse, **caractérisé en ce que**
a) la phase huileuse contient 2 à 10 % en poids par rapport au poids de la phase huileuse, une cire de polyéthylène non ramifiée présentant un poids moléculaire de l'ordre de 400 à 1500 daltons, et au moins une autre cire ou huile ou un mélange de celles-ci ;
b) la phase aqueuse contient 0,5 à 5 % en poids d'un agent filmogène pour la phase aqueuse, choisi dans le groupe comprenant le PVP/PVP-VA, le vinylcaprolactam/ vinylpyrrolidone/vinylimidazol quaternisé (polyquaternium-46) et leurs mélanges, les pourcentages se rapportant au poids total du mascara ;
c) en outre, 0,5 à 20 % en poids d'une phase filmogène externe sont contenus avec un agent filmogène hydrosoluble, choisi dans le groupe comprenant le copolymère PEG/PPG-25/25 diméthicone/acrylate/t-butyle acrylate, le polyuréthane-1 et leurs mélanges, les pourcentages se rapportant au poids total du mascara ; et
d) d'autres pigments cosmétiques, véhicules et adjuvants jusqu'à 100 % en poids.

2. Mascara extensible selon la revendication 1, **caractérisé en ce que** la phase huileuse a une valeur de pénétrabilité à l'aiguille de 5 à 20 dynes/mm à 25° C.

3. Composition de mascara selon la revendication 1,
**caractérisée en ce que** la cire de polyéthylène non ramifiée a un poids moléculaire de l'ordre de 400 à 600 daltons.

4. Composition de mascara selon la revendication 1, **caractérisée en ce que** la phase huileuse contient en outre des cires naturelles, des cires synthétiques, des plastifiants, des esters d'acides gras en C₁₂ à C₂₀ supérieurs, des émulsifiants, des conservateurs et leurs mélanges.

5. Mascara extensible selon la revendication 4, **caractérisé en ce que** les cires sont choisies parmi la cire de candelilla, la cire d'abeille, d'acide stéarique, le monostéarate de glycéryle et leurs mélanges.

6. Mascara extensible selon la revendication 1, **caractérisé en ce que** la proportion de phase huileuse est de 10 à 40 % en poids par rapport au poids total du mascara.

7. Mascara extensible selon la revendication 1, **caractérisé en ce que** la proportion de copolymère PEG/PPG-25/25 diméthicone/acrylate/t-butyl-acrylate est de 0,5 à 10 % en poids par rapport au poids total du mascara.

8. Mascara extensible selon la revendication 1, **caractérisé en ce que** la phase aqueuse contient 0,01 à 2 % en poids d'un extrait aqueux d'algue rhodophycée.

9. Mascara extensible selon la revendication 1, **caractérisé en ce qu'**il présente une viscosité de l'ordre de 50 000 à 500 000 Pa.s, mesurée selon la méthode de Brookfield avec les broches TC/TD/TE à 25° C.

10. Procédé de production d'un mascara extensible, **caractérisé en ce qu'**après le mélange de la phase huileuse et de la phase aqueuse, la phase filmogène externe est ajoutée, l'addition du copolymère PEG/PPG-25/25 diméthicone/acrylate/t-butyl-acrylate s'effectuant de 40 à 76° C et par portion.
